# EUROPÄISCHE PATENTSCHRIFT

(11) **EP 2 316 340 B1**
(45) Veröffentlichungstag und Bekanntmachung des Hinweises auf die Patenterteilung: **06.05.2020**
(21) Anmeldenummer: 10188950.9
(22) Anmeldetag: 26.10.2010
(51) Int. Cl.: A61B 5/18, A61B 5/0428, B60W 40/08

(54) **Signalerfassungsvorrichtung zur Erfassung eines Differenzsignals für eine elektrische Messung eines Vitalparameters eines Lebewesens und Verfahren**
Signal recording device for recording a difference signal for the electrical measurement of a vital parameter of a living being and method
Dispositif de détection de signal pour la détection d'un signal différentiel pour une mesure électrique d'un paramètre vital d'un être vivant et procédé

(30) Priorität: 27.10.2009 DE 102009050755
(43) Veröffentlichungstag der Anmeldung: 04.05.2011
(60) Teilanmeldung: 12156557.6
(73) Patentinhaber: Fraunhofer-Gesellschaft zur Förderung der Angewandten Forschung e.V., 80686 München (DE)
(72) Erfinder: Ershov, Sergey, 91054, Erlangen (DE); Mörsdorf, Hans-Joachim, 90763, Fürth (DE); Rybalko, Ruslan, 90480, Nürnberg (DE); Couronné, Robert, 91058, Erlangen (DE)
(74) Vertreter: Burger, Markus

(56) Entgegenhaltungen:
- EP-A1- 1 611 844
- WO-A2-2008/005478
- DE-A1-102004 036 119
- DE-T2- 69 727 755
- JP-A- 2009 050 679
- JP-A- 2009 142 575
- JP-U- H0 558 105
- KR-A- 20080 038 512
- US-A1- 2005 010 121
- US-A1- 2006 009 691
- US-A1- 2008 243 013
- US-A1- 2010 041 975

## Beschreibung

### Hintergrund der Erfindung

Die vorliegende Erfindung bezieht sich auf eine Signalerfassungsvorrichtung zur Erfassung eines Differenzsignals für eine elektrische Messung eines Vitalparameters eines Lebewesens, beispielsweise eine EKG-Messung, eine Elektrodenanordnung, welche beispielsweise in einem Kfz angeordnet sein kann, und ein Verfahren.

Das Herz als eines der wichtigsten Organe des menschlichen Organismus bildet in der Medizin eine breite Basis zur Diagnose diverser Krankheiten. Durch die räumliche Ausbreitung der Erregung im Herzen entstehen auf der Körperoberfläche elektrische Potentiale. Die Erfassung der zeitlichen Änderungen von Potentialdifferenzen zwischen definierten Stellen an der Körperoberfläche wird als Elektrokardiographie bezeichnet. Die resultierende Aufzeichnung der Potentialverläufe wird Elektrokardiogramm oder auch abgekürzt EKG genannt.

Zur Erfassung des EKG werden über Elektroden auf der Körperoberfläche die elektrischen Potentiale hochohmigen Verstärkern zugeführt. Die aufzunehmenden Potentiale haben eine Amplitude von 50 µV - 5 mV und weisen Frequenzkomponenten im Bereich von 0,1 bis 150 Hz auf. Standardmäßig wird das EKG über Elektroden am Brustkorb bzw. den Armen abgenommen. Je nach Anzahl der aufzuzeichnenden Kanäle schwankt die Zahl der Elektroden typischerweise zwischen drei und zehn.

Derzeit existieren verschiedene Formen eines EKG, wobei sich diese besonders in ihrer Anwendungszeit unterscheiden. Kurzzeit-EKGs dienen einer schnellen Diagnose der Herzfunktion, während Langzeit-EKGs die Herzfunktion über einen längeren Zeitraum aufzeichnen können. Im Gegensatz zu Körperfunktionsüberwachungsgeräten, welche auch einem Endanwender zur Verfügung stehen, beispielsweise Pulsuhren, werden Elektrokardiographen zur Aufnahme von EKGs aufgrund ihrer Komplexität oft nur von Ärzten bedient. Hierbei werden Elektroden an dem Körper eines Menschen nach einem vorgegebenen Prinzip angebracht, über die dann die Potentialdifferenzen gemessen werden können. Die einzelnen am Körper angebrachten Elektroden sind über Kabel mit einer Auswerteeinrichtung verbunden, welche die Änderungen der Potentialdifferenzen über die Zeit erfasst.

Zur Erhöhung der Verkehrssicherheit sind bereits mehrere Systeme bekannt, die auf Basis von im Fahrzeug gemessenen Vitalparametern des Fahrers eine Abschätzung seines psychischen und physischen Zustands erlauben. Die Rückschlüsse solcher Systeme finden Anwendung auf folgenden Gebieten: Diagnose und/oder Therapieunterstützung, Erkennung von Emotionen, Fahrerkonzentration, Müdigkeit und Schläfrigkeit sowie der Quantifizierung von vegetativen Stressniveaus.

Einige Systeme bieten auch die Möglichkeit, in Abhängigkeit vom festgestellten Fahrerzustand auf die Steuerungseinheiten des Automobils einzuwirken. Diese Systeme werden in der Regel wie folgt benannt: Gesundheitsüberwachungssysteme, Fahrerassistenzsysteme, Körperbefindlichkeitsmesseinrichtungen, Fahrzeugsicherheitsvorrichtungen und therapeutische Systeme.

Die DE 102 49 415 B3 beschreibt ein System zur Unterstützung einer Diagnose, Therapie und/oder prophylaktischen Behandlung an einer im Fahrzeug befindlichen Person. Die sensorische Überwachung des Patienten besteht hier in Messung von Gewicht und Respirationsrate.

Die WO 98/25520 beschreibt eine Sicherheitsvorrichtung zur Detektion, Warnung und Verhinderung des Nachlassens der Konzentration des Fahrzeuglenkers und zur Warnung bzw. Verhinderung seiner Schläfrigkeit. Die genannte Schrift schlägt auch vor, ein Emotions-Detektor-System zu implementieren. Die physikalischen Messgrößen hierfür sind Herzrate, Herzratenvariabilität und Körpertemperatur, die mittels Elektroden am Lenkrad abgetastet werden, und Puls, der mittels Pulsmessgeräten an der Oberfläche des Lenkrades abgetastet wird.

Die WO 02/096694 A1 beschreibt ein Verfahren und eine Einrichtung zur Charakterisierung des Zustandes des Fahrers eines Kraftfahrzeuges, wobei anhand der ermittelten Ergebnisse die Steuerungseinheiten des Kraftfahrzeuges beeinflusst werden. Das Konzept gemäß der genannten Veröffentlichung betrachtet gleich mehrere physiologischen Zustandsgrößen wie EKG, Herzrate, EEG, Blutdruck, Hauttemperatur, Hautleitfähigkeit, Augenlidfrequenz, Griffkraft und Bewegungen des Fahrers im Sitz.

Die DE 10 2005 007 963 A1 beschreibt ein Verfahren und eine Einrichtung zur Quantifizierung von vegetativen Stressniveaus. Die genannte Schrift beschreibt das vegetative Stressniveau u. a. auch unter Automobilbedingungen mittels Herzschlagratenüberwachung zu bestimmen. Die Aufnahme der Herzschlagrate kann in diesem Fall über Sensoren oder Sensorfelder, die in die Bedieneinrichtungen - Lenkrad, Schaltknüppel - eines Fahrzeuges integriert sind, erfolgen.

Die DE 60124971 T2 beschreibt ein Gesundheitsüberwachungssystem. Das vorgeschlagene System ist unter anderem für die Überwachung der Person im Fahrzeug vorgesehen. Als Messgrößen werden hier die Herzschlagdaten, aufgenommen von Blechelektroden am Lenkrad, Schweißsekretion von Schweißsensoren (Begriff der Erfinder), aufgenommen am Lenkrad, und das Videobild einer Kamera verwendet. Das Gesundheitsüberwachungssystem dafür optimiert, mehrere Personen gleichzeitig zu überwachen.

Die GB 2 390 460 A beschreibt einen Müdigkeitswarner, der aufgrund von Körpertemperatur, Pulsrate, Blutdruck und Sauerstoffsättigung des Blutes des Fahrers seinen Ermüdungsgrad bestimmt.

Die DE 10 2004 036 119 B4 beschreibt ein Fahrerassistenzsystem zur Müdigkeitserkennung und Aufmerksamkeitsbeurteilung eines Fahrers anhand seiner Pulsfrequenz. Die Sensoren zur Frequenzdetektion des Pulses sollen gemäß der genannten Schrift im Lenkrad, Gangwahlhebel oder Sitz integriert werden.

Die DE 602 14 312 T2 beschreibt ein Gerät zur Messung einer Körperbefindlichkeit des Fahrers. Dafür werden folgende Vitalparameter erfasst: Puls, Schweißsekretion, Hautwiderstand, Atmung, Herzschlagrate und Herzratenvariabilität. Als Ausführungsbeispiele sind folgende Varianten:
a) Körperbefindlichkeitsmesseinrichtung zur Bestimmung, ob der Bediener sich in einem "stabilen Zustand" befindet,
b) Körperbefindlichkeitsmesseinrichtung und ihre Interaktionen mit einem Navigationssystem, und
c) Körperbefindlichkeitsmesseinrichtung und ihre Interaktionen mit einer Musikanlage.

Ein Nachteil vieler diagnostischer Methoden des Herz-Kreislauf-Systems beim Einsatz unter Automobilbedingungen, aber auch bei Diagnostik, beispielsweise bei einem Arzt, besteht darin, dass diese entweder invasiv sind oder dass deren Anwendungsteile (Elektroden und deren Zuleitungen) vor der Messung am Körper angelegt und an eine Signalauswerteelektronik angeschlossen werden müssen.

Die meisten bekannten Systeme zur Fahrerüberwachung mittels Vitalsensorik (beispielsweise Pulsmessungen oder EKG) sind zu allgemein beschrieben und missachten solch wichtige Aspekte wie: Signalqualität, Abdeckung des Fahrgastraums mit Sensoren, Wahrscheinlichkeit und Häufigkeit der Berührung der Sensoren durch den Fahrer, Ablenkung des Fahrers durch die Interaktion mit dem Messsystem und eventuell resultierende Beeinträchtigung der Fahrsicherheit, und Einschwingdauer der Sensoren, d. h. wie schnell nach der Berührung können die Sensoren valide Daten liefern.

Einige Gesundheitsassistenzsysteme versprechen für den Einsatz im Automobil die Möglichkeit einer Diagnosestellung bzw. Diagnoseunterstützung, eine prophylaktische Behandlung oder sogar Therapieunterstützung. Bisher sind aber keine Systeme auf dem Markt bekannt, die im Automobil die Vitaldaten tatsächlich mit der erforderlichen Präzision für die Diagnostik und Therapieunterstützung aufzeichnen können. Dies erklärt sich durch die herausfordernden Randbedingungen bzw. die fehlenden Akzeptanz seitens des Fahrers (Sensor am Körper) in der Automobilumgebung.

Mögliche Systeme zur Erfassung eines Vitalparameters eines Lebewesens, beispielsweise eines EKG, können über eine Mehrzahl von Elektroden verfügen, wobei die Mehrzahl der Elektroden Differenzsignale bilden und daraus ein EKG ableitbar ist. Die Qualität des EKG ist damit stark von der Signalstärke der Elektroden abhängig.

Die US 2008/0243013 A1 beschreibt eine Anordnung von Elektrodeneinheiten an einem Lenkrad. Mithilfe einer Impedanzmesseinheit wird eine Impedanz zwischen einer linken und einer rechten Elektrode gemessen, um die Elektroden zu finden, welche einen Fahrzeugbenutzer kontaktieren. Sobald eine Kombination von Elektroden gefunden ist, die den Fahrzeugbenutzer kontaktieren, so kann ein elektrokardiographisches Signal gemessen werden.

Die Druckschrift US 2005/0010121 zeigt Verfahren und Systeme zur Detektion eines Signals beschreibend zumindest einen Herzschlag, eine Herzrate oder einen EKG eines Tiers. Die Verfahren und Methoden umfassen das Scannen jeder einer Mehrzahl von Elektroden hinsichtlich eines Signals, welches darauf hinweist, dass die Elektrode ein Tier kontaktiert und, das Auswählen eines Signals von jeder zumindest eines Paares von Elektroden, wobei jede ausgewählte Elektrode ein Signal aufweist, welches beschreibt, dass die Elektrode Kontakt mit dem Lebewesen hat.

Die Druckschrift WO 2008/005478 zeigt medizinische Geräte zur Vorbeugung oder Behandlung von neurologischen Unregelmäßigkeiten wie eine elektrische Stimulation.

Die Druckschrift US 2010/0041975 A1 zeigt verschiedene Systeme und Verfahren zum Überwachen physiologischer Daten eines Patienten.

Die Druckschrift JP 2009142575 zeigt eine Anordnung von Elektroden in einem Kfz, wobei an einem Lenkrad des Kfz eine linke Elektrode und eine rechte Elektrode angeordnet sind. Ferner befindet sich in einem Fahrzeugsitz eine Elektrode.

Die Druckschrift DE 10 2004 036 119 A1 zeigt ein Fahrerassistenzsystem zur Müdigkeitserkennung bzw. Aufmerksamkeitsbeurteilung eines Fahrzeugführers, bei dem Elektroden sowohl in einem Lenkrad, einem Schaltknopf als auch einem Sitz untergebracht werden können, um durch diese Elektroden eine Pulsfrequenz des Fahrzeugführers zu ermitteln.

Dokument KR20080038512 beschreibt eine Signalerfassungseinrichtung zur EKG Messung eines Fahrzeuginsassen.

Die Aufgabe der vorliegenden Erfindung ist es, eine Vorrichtung zu schaffen, die eine verbesserte Aufnahme eines Vitalparameters eines Lebewesens ermöglicht.

### Zusammenfassung der Erfindung

Eine Signalerfassungsvorrichtung gemäß Anspruch 1 und ein Verfahren gemäß Anspruch 13 lösen diese Aufgabe.

Die vorliegende Erfindung schafft eine Signalerfassungsvorrichtung mit einer Mehrzahl von Elektroden und einer Elektrodenauswahlvorrichtung, die ausgebildet ist um ein Paar von Elektroden aus der Mehrzahl von Elektroden auszuwählen, so dass ein geeignetes Differenzsignal zur Messung eines Vitalparameters eines Lebewesens ableitbar ist.

Der Kerngedanke der vorliegenden Erfindung basiert auf der Erkenntnis, dass eine verbesserte Aufnahme eines Vitalparameter eines Lebewesens ermöglicht wird, wenn eine Signalerfassungsvorrichtung über eine Mehrzahl von Elektroden, typischerweise mehr Elektroden als zur Bereitstellung eines Differenzsignal zwingend erforderlich sind, verfügt und wenn eine Elektrodenauswahlvorrichtung der Signalerfassungsvorrichtung abhängig von den Elektrodensignalen ein Paar von Elektroden aus der Mehrzahl der Elektroden auswählen kann, bei welchem ein zur Messung des Vitalparameters geeignetes Differenzsignal ableitbar ist, da so ein unterschiedlich guter bzw. sogar bei einigen Elektroden vollständig fehlender Kontakt zwischen den Elektroden und dem Lebewesen nicht zu einem unbrauchbaren Messergebnis führt, sondern vielmehr zu einer automatischen Auswahl geeigneter Elektroden. Ausgehend davon, dass mindestens ein Teil der Mehrzahl von Elektroden das Lebewesen kontaktiert, wird aufgrund der Auswahl eines Paares von Elektroden, basierend auf den Elektrodensignalen, ein Differenzsignal mit einer ausreichende guten Signalqualität zur Messung des Vitalparameters verwendet.

Ein Vorteil der vorliegenden Erfindung ist somit, dass durch die Mehrzahl von Elektroden eine Redundanz von Elektroden, die den Körper kontaktieren, entsteht, und dass durch die Elektrodenauswahlvorrichtung das Paar von Elektroden ausgewählt werden kann, welches das geeignetste Differenzsignal zur Messung des Vitalparameters besitzt.

Weiterhin schafft die vorliegende Erfindung eine Elektrodenanordnung mit einer Mehrzahl von Elektroden, wobei die Mehrzahl der Elektroden in einem Kraftfahrzeug angeordnet ist, um den Benutzer zu kontaktieren, und wobei mind. eine der Elektroden in einem Bedienelement des Kraftfahrzeug angeordnet ist und mind. eine weitere der Elektrodein in einem Ruheelement des Kraftfahrzeug angeordnet ist.

Im Folgenden wird unter einem Bedienelement auch ein Steuerelement wie beispielsweise ein Lenkrad oder ein Schaltknauf oder ein Schaltelement wie beispielsweise ein Drehschalter eines Radios oder ein Blinkerhebel verstanden.

Ein "Kraftfahrzeug" wird im Folgenden auch kurz als "Kfz" bezeichnet.

Dieser Aspekt der vorliegenden Erfindung basiert auf der Erkenntnis, dass ein Kfz-Benutzer während der Fahrt typischerweise seine Hände nicht dauerhaft an Bedienelementen des Kfz bzw. am Lenkrad hat. Ausgehend von der Erkenntnis, dass ein Kfz-Benutzer während der Fahrt oftmals eine seiner Hände auf einem Bedienelement, beispielsweise Lenkrad oder Schalthebel des Kfz, und eine andere Hand auf einem Ruheelement, beispielsweise Armauflagefläche des Kfz, hat, ist aufgrund der Integration mindestens einer Elektrode in ein Ruheelement des Kfz eine erhöhte Kontaktierungswahrscheinlichkeit und damit eine verbesserte kontinuierliche Erfassung für die elektrische Messung eines Vitalparameters des Kfz-Benutzers gegeben.

Ein weiterer Vorteil der vorliegenden Erfindung ist somit, dass Elektrodensignale auch geliefert werden, wenn ein Kfz-Benutzer nicht beide Hände an einem Bedienelement, beispielsweise Lenkrad, hat.

### Figurenkurzbeschreibung

Bevorzugte Ausführungsbeispiele der vorliegenden Erfindung werden nachfolgend Bezug nehmend auf die beiliegenden Zeichnungen näher erläutert. Es zeigen:
- Fig. 1: eine schematische Darstellung einer Signalerfassungsvorrichtung gemäß einem ersten Ausführungsbeispiel nicht Teil der vorliegenden Erfindung;
- Fig. 2: ein Bild einer Elektrodenanordnung gemäß einem zweiten Ausführungsbeispiel nicht Teil der vorliegenden Erfindung;
- Fig. 3: eine schematische Darstellung einer Signalerfassungsvorrichtung gemäß einem dritten Ausführungsbeispiel nicht Teil der vorliegenden Erfindung;
- Fig. 4: eine schematische Darstellung einer Signalerfassungsvorrichtung gemäß einem vierten Ausführungsbeispiel der vorliegenden Erfindung;
- Fig. 5: ein Bild einer Elektrodenanordnung gemäß einem fünften Ausführungsbeispiel der vorliegenden Erfindung;
- Fig. 6: ein Bild einer Elektrodenanordnung gemäß einem sechsten Ausführungsbeispiel der vorliegenden Erfindung; und
- Fig. 7: ein Bild eines Bedienelements zur Verwendung in einem Ausführungsbeispiel der vorliegenden Erfindung; und
- Fig. 8: ein Flussdiagramm eines Verfahren gemäß einem Ausführungsbeispiel der vorliegenden Erfindung.

### Detaillierte Beschreibung der Ausführungsbeispiele

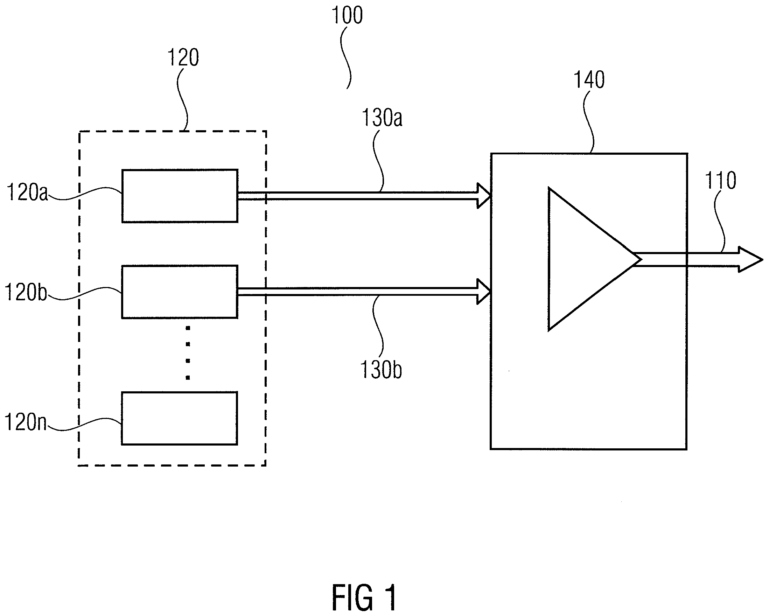

Fig. 1 zeigt eine schematische Darstellung einer Signalerfassungsvorrichtung 100 gemäß einem ersten Ausführungsbeispiel nicht Teil der vorliegenden Erfindung. Die Signalerfassungsvorrichtung 100 zur Erfassung eines Differenzsignals 110 für eine elektrische Messung eines Vitalparameters eines Lebewesens umfasst eine Mehrzahl 120 von Elektroden 120a-120n und eine Elektrodenauswahlvorrichtung 140. Die Elektroden 120a-120n sind mit der Elektrodenauswahlvorrichtung 140 verbunden, um Elektrodensignale 130 an die Elektrodenauswahlvorrichtung 140 zu liefern.

Elektroden 120a-120n aus der Mehrzahl von Elektroden können das Lebewesen kontaktieren und an die Elektrodenauswahlvorrichtung 140 Elektrodensignale 130 liefern. Basierend, beispielsweise auf der Stärke (bzw. Amplitude) der Elektrodensignale 130 (oder einer anderen charakteristischen Eigenschaft der Elektrodensignale 130, wie etwa einer Intensität und charakteristischen Signalform oder eines charakteristischen Frequenzanteils) der einzelnen Elektroden 120a-120n kann die Elektrodenauswahlvorrichtung 140 ein Paar von Elektroden 120a-120n auswählen, welches eine Bereitstellung eines am besten geeigneten Differenzsignals 110 oder zumindest ausreichend gut geeigneten Differenzsignals 110 zur Messung des Vitalparameters ermöglicht und daraus das Differenzsignal 110 zur Messung des Vitalparameters des Lebewesens ableiten. Durch die Auswahl des Paares von Elektroden 120a-120n, basierend auf der Signalstärke der Elektrodensignale 130, kann somit gewährleistet werden, dass immer ein bestmöglichstes (oder zumindest ausreichend gutes) Differenzsignal 110 zur Messung des Vitalparameter des Lebewesens verwendet wird. Weiterhin ist die Elektrodenauswahlvorrichtung 140 in der Lage, bei Änderung der Elektrodensignale 130 der Elektroden 120a-120n (oder bei Änderung von charakteristischen Eigenschaften derselben) ein anderes Paar von Elektroden 120a-120n zur Bildung des Differenzsignals 110 auszuwählen, welches ein besseres Differenzsignal 110 als das bisherige Paar von Elektroden 120a-120n bildet (bzw. eine Bereitstellung eines besseren Differenzsignal 110 ermöglicht). Durch Nutzung mehrerer redundanter Elektroden 120a-120n können die Möglichkeiten zur Aufnahme eines Differenzsignals 110 wesentlich erhöht werden. Die Elektroden 120a-120n müssen hierbei nicht zwangsläufig fest an einem Lebewesen befestigt werden, sondern können auch auf Flächen angebracht sein, welche das Lebewesen (dauerhaft oder vorübergehend) berührt. So kann die Signalerfassungsvorrichtung 100 bzw. die Mehrzahl 120 der Elektroden der Signalerfassungsvorrichtung 100 beispielsweise in einem Kfz angeordnet sein.

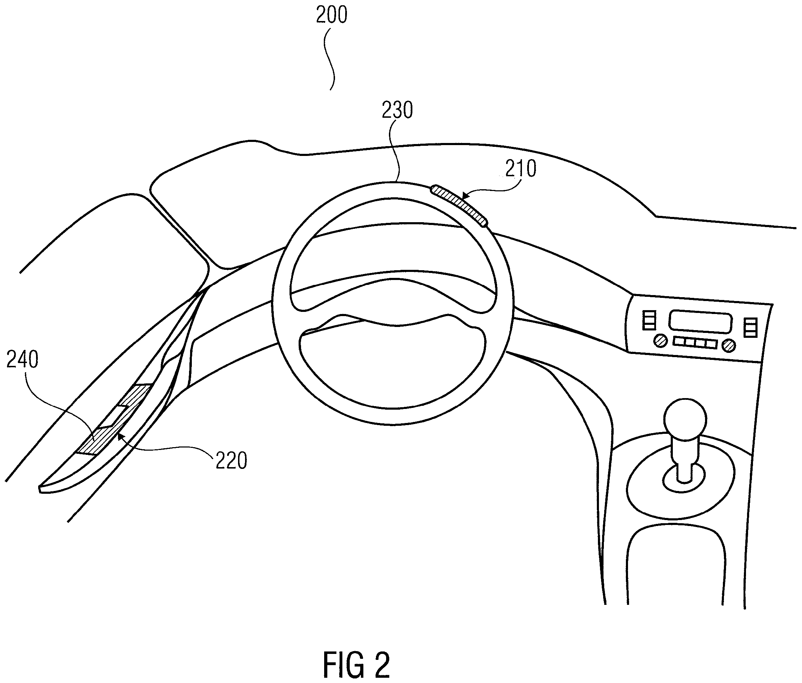

Fig. 2 zeigt ein Bild einer Elektrodenanordnung 200 gemäß einem zweiten Ausführungsbeispiel nicht Teil der vorliegenden Erfindung. Die Elektrodenanordnung 200 ist in einem Kfz integriert, wobei eine erste Elektrode 210 in, an oder auf einem Bedienelement 230 angebracht ist. Eine zweite Elektrode 220 ist in, an oder auf einem Ruheelement 240 des Kfz angebracht. Das Bedienelement 230 kann beispielsweise ein Lenkrad des Kfz sein, das Ruheelement 240 beispielsweise eine Armauflagefläche der Fahrertür des Kfz sein.

Die erste Elektrode 210 und die zweite Elektrode 220 sind so im Kfz angeordnet, dass sie den Kfz-Benutzer kontaktieren können (beispielsweise, wenn dieser sich in einer Fahrposition befindet). Beispielsweise kann die erste Elektrode 210 so angeordnet sein, dass sie die rechte Hand des Kfz-Benutzers kontaktiert, und die zweite Elektrode 220 so angeordnet sein, dass sie die linke Hand des Kfz-Benutzers kontaktiert. Die gezeigte Elektrodenanordnung 200 kann damit genutzt werden, um beispielsweise ein Differenzsignal zur elektrischen Messung eines Vitalparameters des Kfz-Benutzers, beispielsweise EKG, zu erfassen. Hierbei kann ein Differenzsignal zwischen der ersten Elektrode 210 und der zweiten Elektrode 220 (beispielsweise in Form eines Differenzsignals, dass eine Differenz der an den Elektroden anliegenden Potentiale beschreibt) erfasst werden, wobei das Differenzsignal durch die vom Herzschlag des Lebewesens erzeugten Potentiale entsteht.

Weiterhin ist es möglich, dass zusätzliche Elektroden in oder an dem Bedienelement 230 sowie in oder an weiteren Bedienelementen oder Ruheelementen des Kfz angeordnet sind.

Eine Elektrode wird im Folgenden auch als Messelektrode oder Kontaktelektrode bezeichnet.

Ein Kfz-Benutzer wird im Folgenden auch kurz als Fahrer bezeichnet.

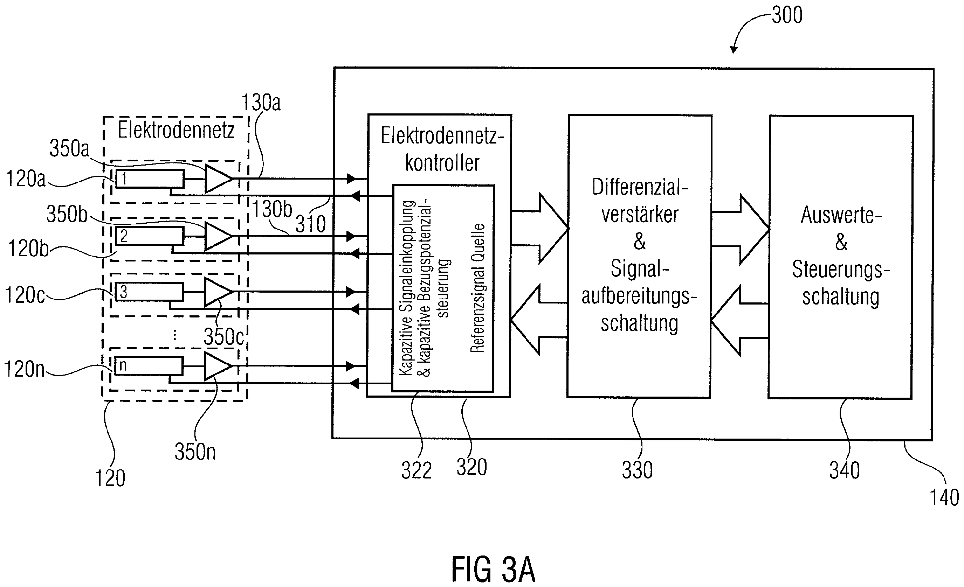

Fig. 3a zeigt eine schematische Darstellung einer Signalerfassungsvorrichtung 300 gemäß einem dritten Ausführungsbeispiel nicht Teil der vorliegenden Erfindung. Die Signalerfassungsvorrichtung 300 umfasst eine Mehrzahl von Elektroden 120a-120n bzw. ein Elektrodennetz 120. Das Elektrodennetz 120 umfasst mind. eine ersten Elektrode 120a, eine zweite Elektrode 120b und eine dritte Elektrode 120c. Das Elektrodennetz 120 kann beliebig viele weitere Elektroden 120a-120n umfassen. Weiterhin umfasst die Signalerfassungsvorrichtung 300 eine Elektrodenauswahlvorrichtung 140. Die Elektrodenauswahlvorrichtung 140 umfasst einen Elektrodennetzcontroller 320, eine Differentialverstärker- und Signalaufbereitungsschaltung 330 und eine Auswerte- und Steuerungsschaltung 340. Der Elektrodennetzcontroller 320 umfasst eine Einrichtung 322 zur kapazitiven Signaleinkopplung und kapazitiven Bezugspotentialansteuerung mit einer Referenzsignalquelle. Mit anderen Worten gesagt zeigt Fig. 3a die beispielhafte Integration der Signalerfassungsvorrichtung 300 bzw. des Systems zur Erfassung des EKG mittels eines Netzes 120 der Kontaktelektroden 120a-120n oder kapazitiven Elektroden 120a-120n mit kapazitiver Einkopplung des Signals und kapazitiver Bezugspotentialsteuerung 322. Die Elektroden 120a-120n umfassen jeweils einen nachgeschalteten Verstärker 350a-350n. Die Elektroden 120a-120n sind mit dem Elektrodennetzcontroller so verbunden, dass sie Elektrodensignale 130a-130n an den Elektrodennetzcontroller 320 liefern können. Die Einrichtung 322 des Elektrodennetzcontrollers 320 kann ihrerseits Signale 310 an die Elektroden 120a-120n liefern. Die EKG-Elektroden 120a-120n sind beispielsweise im Fahrgastraum eines Kfz integriert, wie dies beispielsweise anhand der Fig.2 beschrieben wurde. Die Schaltung verfügt über keine separate Referenzelektrode zur Bezugspotentialsteuerung und Signalgleichtaktunterdrückung.

Die Signale der Bezugspotentialsteuerung (Stabilisierung der Nulllinie) und Signalgleichtaktunterdrückung werden dem Kfz-Benutzer über dieselben Messelektroden 120a-120n, welche zur EKG-Abtastung verwendet werden, zugeführt. Dafür ist eine spezielle Schaltung zur kapazitiven Einkopplung des EKG-Messsignals und kapazitiven Rückkopplung des Bezugssignals am Eingang des Messsystems vorgesehen, wie beispielsweise die Einrichtung 322. Genauso wird hier zusätzlich über die Messelektroden 120a-120n ein Referenzsignal 310 (ein sinusförmiges oder moduliertes Signal) zwecks Elektrodenkontrolle (Ermittlung der den Kfz-Benutzer kontaktierenden Elektroden 120a-120n) dem Kfz-Benutzer bzw. Fahrer zugeführt. Der Amplituden- und Frequenzbereich dieses Referenzsignals 310 entspricht den zugelassenen Grenzwerten für EKG-Messsysteme (z. B. Patientenableitströme gemäß ISO 60601). Dabei wird das Referenzsignal 310 zur Elektrodenkontrolle nicht an allen Messelektroden 120a-120n gleichzeitig eingekoppelt, sondern wird schrittweise einer nach der anderen Messelektrode 120a-120n zugeführt. Somit kann iterativ eine Suchroutine gestartet werden, die andere Elektroden 120a-120n (beispielsweise andere als die Elektrode, an der gerade das Referenzsignal 310 eingekoppelt wird) mit bestehendem Kontakt zum Kfz-Benutzer ermittelt und die bevorzugt gleichzeitig eine Aussage darüber liefert, ob die Elektrode, an der das Referenzsignal 310 eingekoppelt wird in ausreichend gutem Kontakt mit dem Kfz-Benutzer steht. Liefert die Suchroutine keine Ergebnisse, wird also an keiner von der Quellelektrode (Elektrode, an der das Referenzsignal 310 eingekoppelt ist) verschiedenen Elektrode ein Elektrodensignal basierend auf dem Referenzsignal 310 ermittelt, bedeutet das, dass kein Kontakt zur initialen Quellelektrode besteht. Es wird dann das Referenzsignal 310 zur Elektrodenkontrolle einer anderen Messelektrode 120a-120n als Quellelektrode zugeführt. Die Elektrodenerkennungsroutine wird die Suche nach dem Kontakt mit Elektroden 120a-120n nach einer vordefinierten Reihenfolge starten, somit wird die Suchzeit minimiert und die Elektrodenerkennungsroutine zu einem systematischen Prozess. Die Reihenfolge der Zuführung des Referenzsignals 310 ergibt sich aus der Wahrscheinlichkeit, mit der die entsprechende Elektrode 120a-120n durch den Fahrer berührt wird (Kontaktierungswahrscheinlichkeit). So empfiehlt sich beispielsweise eine linke (EKG-)Elektrode 120a-120n am Lenkrad des Kfz als der Startpunkt der Elektrodenerkennungsroutine.

Die Suchroutine selbst funktioniert folgendermaßen: mittels des in dem Elektrodennetzcontroller 322 eingebauten Komparatormesssystems werden die vorverstärkten Signale 130a-130n von einzelnen Elektroden 120a-120n erfasst und mit einem initialen Referenzsignal (bzw. in manchen Ausführungsbeispielen sogar mehreren initialen Referenzsignalen), welches (bzw. welche) verschiedene mögliche Signalformen, beispielsweise sinusförmig oder moduliert aufweisen kann (bzw. können) verglichen. Somit werden die Elektroden 120a-120n mit bestehendem Kontakt zum Fahrer ermittelt und auf Basis der Signalstärke der Elektrodensignale 130a- 130n die Elektroden 120a-120n mit der besten Signalqualität ausgewählt. Danach werden die ausgewählten Elektroden 120a-120n über Multiplexermatrizen mit verschiedenen logischen Algorithmen dem Differentialverstärker 330 zugeführt. Letztendlich wird das Paar der (EKG-)Elektroden 120a-120n zur Erfassung des EKG ausgewählt, welches den besten Kontakt zum Kfz-Benutzer hat und das beste Differenzsignal 110 liefert. Das Referenzsignal 310, das für die Elektrodendetektion (Kontakt zum Fahrer, Signalqualität) verwendet wird, wird später aus dem Nutzsignal ausgefiltert.

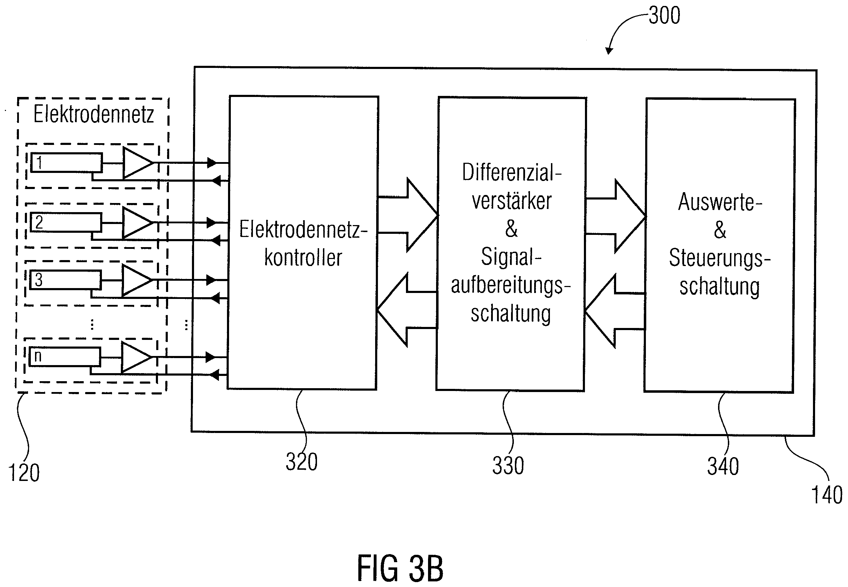

Fig. 3b zeigt die Signalerfassungsvorrichtung 300 ohne die Einrichtung 322 zur kapazitiven Signaleinkopplung und kapazitiven Bezugspotentialansteuerung mit einer Referenzsignalquelle.

Die Messvorrichtung bzw. Signalerfassungsvorrichtung 300 in Fig. 3a und 3b besteht also im Wesentlichen aus dem "Netz" von umschaltbaren aktiven Elektroden 120a-120n, dem Elektrodennetzcontroller 320, dem differentialen Instrumentationsverstärker und der Signalaufbereitungsschaltung 330 und der Auswerte- und Steuerungsschaltung 340. Die gezeigte Signalerfassungsvorrichtung 300 kann beispielsweise in einem Kfz integriert sein, um beispielsweise das EKG eines Fahrers kontinuierlich während der Fahrt aufzuzeichnen. Die Signalerfassungsvorrichtung 300 misst über die Elektroden 120a-120n die Differenz der durch Herzaktivität entstandenen elektrischen Potentiale auf der Körperoberfläche des Fahrers bzw. Kfz-Benutzers. Im ersten Schritt werden Elektroden 120a-120n mit bestehendem elektrischem oder kapazitiv eingekoppelten Kontakt zum Fahrer durch schematische (Elektrodennetzcontroller 320) und algorithmische Verfahren automatisch erkannt und aktiviert. Sich nicht im Gebrauch befindende Elektroden 120a-120n (Elektroden ohne Kontakt zum Kfz-Benutzer) werden abgeschaltet, aber dennoch kontinuierlich in ihrer Signalstärke und Qualität überwacht, um bei Bedarf direkt die beste Elektrodenkonfiguration (das Paar von Elektroden, welches das beste oder ein zumindest hinreichend gutes Differenzsignal liefert) aktivieren zu können. Für die Messung werden bevorzugt mindestens zwei Elektroden 120a-120n aktiviert. Im zweiten Schritt wird das Differenzsignal 110 zwischen den aktiven Elektroden 120a-120n gemessen. Für die nachstehende EKG-Messung wird das Paar von Elektroden 120a-120n mit dem besten Differenzsignal 110 ausgewählt. Damit wird die Abnahme der differentiellen EKG-Ableitung, die der ersten EKG-Ableitung nach Einthoven ähnelt, ermöglicht. Die Elektrodenerkennungsroutine kann je nach Ausführung des Systems entweder im Hintergrund der Messung immer aktiv bleiben und ständig die besten Signalquellen (Elektroden 120a-120n mit bestem Kontakt zum Kfz-Benutzer bzw. Fahrer) suchen, oder aber wird erst nach Abbruch des elektrischen bzw. kapazitiv eingekoppelten Kontaktes mit dem Kfz-Benutzer bzw. Fahrer neu gestartet.

Da zwischen den einzelnen Standardableitungen eines EKG gewisse lineare Abhängigkeiten bestehen, ist es durch Kombination mehrerer Elektroden 120a-120n beispielsweise möglich, eine bestimmte, der Anwendung gemäße, Ableitung (teilweise) zu rekonstruieren, obwohl die tatsächliche Messung momentan nicht möglich ist.

Mit anderen Worten gesagt überwacht die Elektrodenauswahlvorrichtung 140 kontinuierlich die Elektrodensignale 130a-130n der Elektroden 120a-120n und wählt basierend auf den Elektrodensignalen 130a-130n das Paar von Elektroden 120a-120n zur Bestimmung des Differenzsignals 110 aus, welches das beste Differenzsignal 110 liefert.

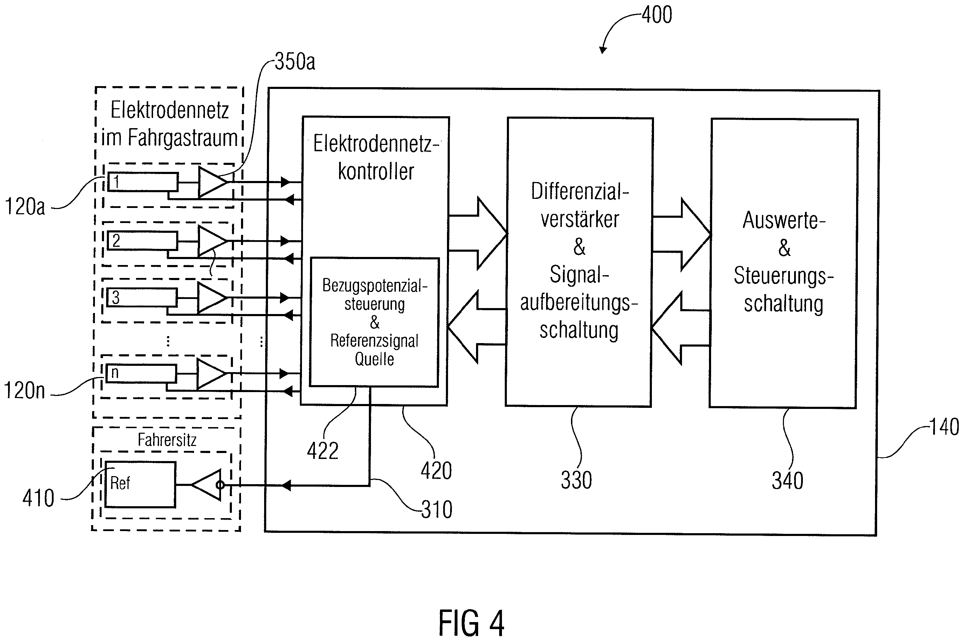

Fig. 4 zeigt eine schematische Darstellung einer Signalerfassungsvorrichtung 400 gemäß einem vierten Ausführungsbeispiel der vorliegenden Erfindung. Die Signalerfassungsvorrichtung 400 ist ausgebildet, um beispielsweise in einem Kfz integriert zu werden, um beispielsweise ein EKG eines Kfz-Benutzers aufzunehmen. Die Signalerfassungsvorrichtung 400 umfasst ein Elektrodennetz 120 mit Kontaktelektroden 120a-120n. Jeder der Kontaktelektroden 120a-120n ist ein Verstärker 350a-350n nachgeschaltet. Ein Ausgang der Verstärker 350a-350n ist jeweils mit einem Eingang der Elektrodenauswahlvorrichtung 140 verbunden. Die Elektrodenauswahlvorrichtung 140 umfasst zusätzlich zu dem Elektrodennetzcontroller 420 eine Differenzverstärker- und Signalaufbereitungsschaltung 330 und eine Auswerte- und Steuerungsschaltung 340. Weiterhin umfasst die Signalerfassungsvorrichtung 400 im Gegensatz zu der Signalerfassungsvorrichtung 300, eine Referenzelektrode 410, welche beispielsweise in einem Fahrersitz des Kfz integriert ist. Die Referenzelektrode 410 ist mit einer Bezugspotentialsteuerung und Referenzsignalquelle 422 des Elektrodennetzcontrollers 420 verbunden.

Mit anderen Worten gesagt zeigt Fig. 4 die beispielhafte Integration der Signalerfassungsvorrichtung 400 bzw. des Systems zur Erfassung des EKG mittels eines Netzes 120 der Kontaktelektroden 120a-120n oder kapazitiven Elektroden 120a-120n mit einer Bezugspotentialelektrode 410 (die in der Medizintechnik auch mit "Driven Right Leg" bezeichnet wird, und die der Bezugspotentialsteuerung dient) im Fahrersitz des Kfz. Die EKG-Elektroden 120a-120n sind im Fahrgastraum des Kfz integriert. Die großflächige Referenz- bzw. Bezugspotentialelektrode 410 zur Bezugspotentialsteuerung und Signalgleichtaktunterdrückung ist im Fahrersitz des Kfz integriert.

Zusätzlich zu den für die EKG-Erfassung standardmäßigen Lösungen wird dem Kfz-Benutzer zur Elektrodenkontrolle (Elektrodenerkennungsroutine) ein zusätzliches Referenzsignal 310 über die Referenzelektrode 410 im Fahrersitz zugeführt. Dieses Signal kann ein DC-Signal, ein sinusförmiges Signal oder ein moduliertes Signal sein. Der Amplituden- und Frequenzbereich dieses Referenzsignals 410 entspricht den zugelassenen Grenzwerten für EKG-Messsysteme (z. B. Patientenableitströme gemäß ISO 60601). Mittels des im Elektrodennetzcontroller 420 der Elektrodenauswahlvorrichtung 140 eingebauten Komparatormesssystems werden die vorverstärkten Signale von einzelnen Elektroden 120a-120n erfasst und mit dem initialen Referenzsignal 310 verglichen. Dadurch werden die Elektroden 120a-120n mit bestehendem Kontakt zum Kfz-Benutzer ermittelt und auf Basis der Signalstärke (beispielsweise des Referenzsignal-bedingten Anteils der Elektrodensignale) die Elektroden 120a-120n mit der besten Signalqualität ausgewählt. Anschließend werden die ausgewählten Elektroden 120a-120n über eine Multiplexermatrix mit verschiedenen logischen Algorithmen dem Differentialverstärker 330 der Elektrodenauswahlvorrichtung 140 zugeführt. Letztendlich wird das Paar der EKG-Elektroden 120a-120n zur Erfassung des EKG ausgewählt, welches den besten Kontakt zum Kfz-Benutzer hat und entsprechend das beste Differenzsignal 110 liefert (bzw. die Bereitstellung des besten Differenzsignals 110 ermöglicht). Die Summe der vorverstärkten Signale von beiden ausgewählten Elektroden 120a-120n bzw. von dem ausgewählten Paar von Elektroden 120a-120n wird vor der Differentialverstärkung zwecks Bezugspotentialsteuerung zusätzlich abgeleitet und an die Referenzelektrode 410 im Fahrersitz des Kfz zugeführt. Das Referenzsignal 310, das für die Elektrodendetektion (Kontakt zum Kfz-Benutzer, Signalqualität) verwendet wird, wird später aus dem Nutzsignal ausgefiltert.

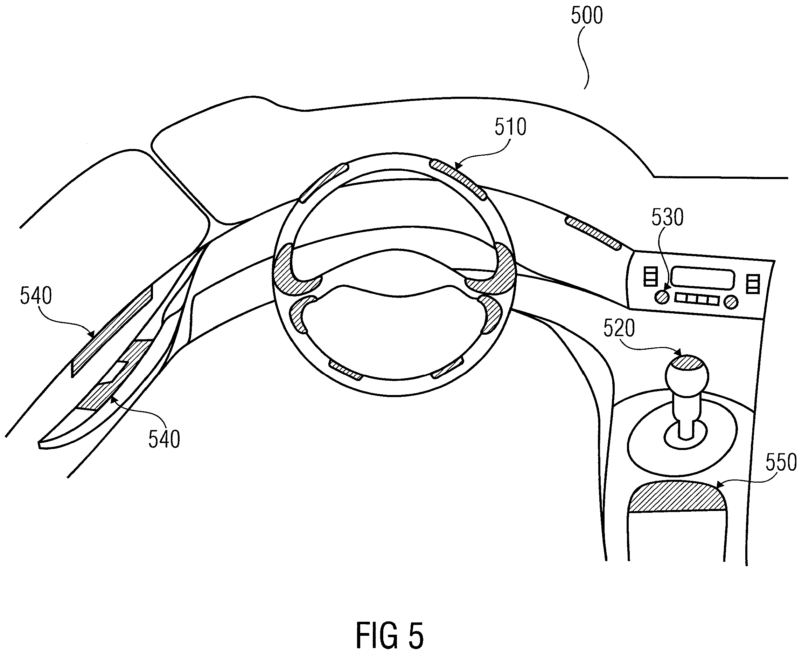

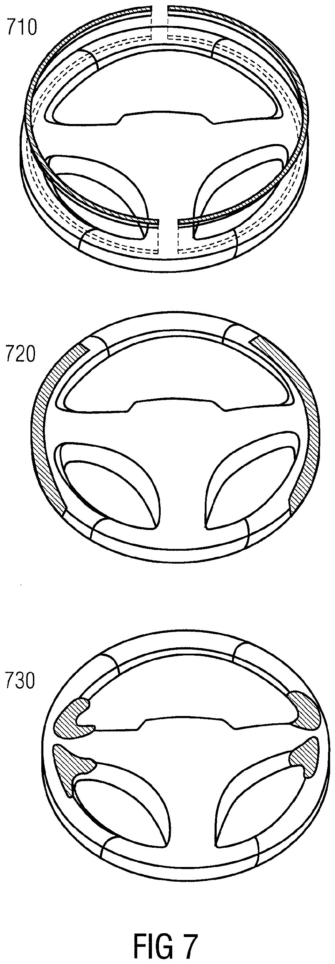

Fig. 5 zeigt ein Bild einer Elektrodenanordnung 500 gemäß einem fünften Ausführungsbeispiel der vorliegenden Erfindung. Die Elektroden der Elektrodenanordnung 500 sind im fünften Ausführungsbeispiel in verschiedenen Bedien- und Ruheelementen des Kfz integriert. Bedienelemente des Kfz können beispielsweise ein Lenkrad 510, eine Bedienkonsole eines Radio- oder Navigationsgerätes 530 oder ein Schaltknauf 520 sein. Ruheelemente können beispielsweise eine Türverkleidung 540 oder eine Mittelarmkonsole 550 sein. Bei dem Lenkrad 510 werden die Differenzsignale erfasst, wenn beide Hände des Fahrers auf dem Lenkrad ruhen, bzw. das Lenkrad greifen. Elektroden werden hier links und rechts in den Lenkradkranz integriert. Drei beispielhafte Lenkräder 510 sind in Fig. 7 gezeigt. Die Elektroden können beispielsweise als Ringelektroden 710, Flächenelektroden 720 oder Kleinflächenelektroden 730 am Lenkrad 510 ausgeführt sein. Bei dem Schaltknauf 520 können die Differenzsignale erfasst werden, wenn beispielsweise eine Hand auf dem Lenkrad 510 und die zweite Hand auf dem Schaltknauf 520 liegen, wobei die Handballenelektrode auf dem Schaltknauf 520 auf der oberen Seite des Schaltknaufs 520 integriert werden kann. An der Bedienkonsole 530 können Differenzsignale bei Bedienung von einem Navigationsgerät oder einem Radio erfasst werden (beispielsweise, wenn die weite Hand gleichzeitig das Lenkrad 510 greift oder auf einer Ablage ruht).

Weiterhin können Ruheelemente zur Erfassung der Differenzsignale genutzt werden. Die Türverkleidung 540 kann zur Erfassung der Differenzsignale genutzt werden, wenn sich eine Hand des Kfz-Benutzers beispielsweise auf dem Lenkrad 510 befindet und die zweite Hand des Kfz-Benutzers auf der Türverkleidung 540 befindet. Die Armlehne 550 kann beispielsweise zur Erfassung der Differenzsignale genutzt werden, wenn sich eine Hand auf dem Lenkrad 510 befindet und die zweite Hand auf der Armlehne 550 befindet. Weiterhin können Elektroden auch in anderen Baugruppen des Kfz, die mit dem Kfz-Benutzer in Kontakt kommen, beispielsweise Fahrersitz, integriert werden.

Da es bei der EKG-Ableitung um die Erfassung der elektrischen Potentiale am Körper des Fahrers geht, müssen bei der Integration der Elektroden in einen Innenraum eines Kfz die Elektroden im Fahrgastraum mit Rücksicht auf potentielle Abtastorte (Steuer-, Bedien-, Schaltelemente bzw. Elemente der Innenausstattung des Kfz, die der Fahrer während der Fahrt unaufgefordert möglichst lang und häufig berührt), Signalqualität und Sicherheitsaspekte integriert werden.

Kontaktierungen des Fahrers mit Elektroden (Berühren der Elektrodenfläche) werden vor allem durch die Kleidung minimiert und passieren vorwiegend über die Hände bzw. Handinnenflächen. Die in Fig. 5 gezeigte Elektrodenanordnung 500 ist auf eine möglichst hohe Kontaktierungswahrscheinlichkeit mit dem Kfz-Benutzer optimiert.

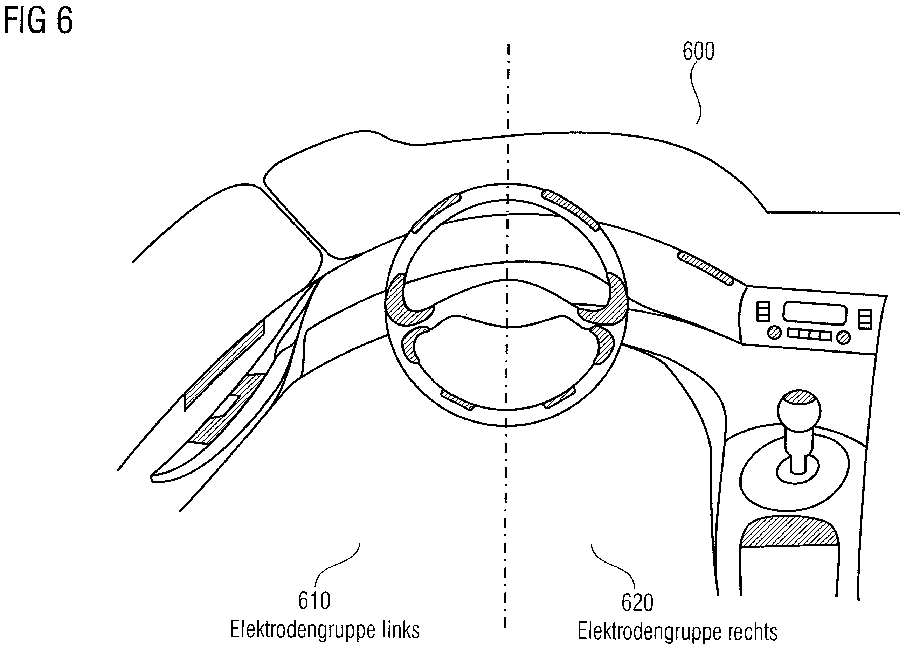

Eine mögliche Erweiterung der in Fig. 5 gezeigte Elektrodenanordnung 500 ist in Fig. 6 als Elektrodenanordnung 600 gemäß einem sechsten Ausführungsbeispiel der vorliegenden Erfindung dargestellt. Die EKG-Elektroden der Elektrodenanordnung im Fahrgastraum können zwecks Optimierung der Elektrodenerkennungsroutine in zwei Gruppen unterteilt werden. So werden die Elektroden, die vorwiegend mit der linken Hand bzw. dem linken Arm des Fahrers in Kontakt kommen (also beispielsweise Elektroden, die links von der Mitte des Fahrersitzes angeordnet sind), als linke Elektrodengruppe 610 definiert und die Elektroden, die vorwiegend mit der rechten Hand bzw. dem rechten Arm des Fahrers in Kontakt kommen (also beispielsweise Elektroden, die rechts von der Mitte des Fahrersitzes angeordnet sind), als rechte Elektrodengruppe 620 definiert.

Die Elektrodenanordnungen 500 und 600 können beispielsweise als das Elektrodennetz 120 der in Figur 3 beschriebenen Signalerfassungsvorrichtung 300 ausgebildet sein.

Als Material für die Elektroden, welche in dem ersten bis sechsten Ausführungsbeispiel genannt sind wird metallisierter Kunststoff bevorzugt. Gegenüber massiven vollmetallischen Elektroden haben die Elektroden aus metallisiertem Kunststoff ein besseres Temperaturverhalten. Bei direkter Sonneneinstrahlung können sich die metallischen Flächen im Fahrgastraum auf über 60°C erhitzen, was der Schmerzgrenze für Temperaturempfindlichkeit des Menschen entspricht. Im Fall von Elektroden aus metallisiertem Kunststoff spielt der Kunststoff die Rolle des thermischen Isolators (bzw. fungiert als thermischer Isolator) und weist darüber hinaus auch eine deutlich geringere thermische Kapazität auf. Die dünne Metallschicht dieser Elektroden wird zwar auch durch die Umgebungsbedingungen stark erhitzt, speichert aber nicht so viel Wärmeenergie in sich und gleicht diese sehr schnell mit der Körperwärme bei Berührung ab.

Der Einsatz der massiven vollmetallischen Elektroden wird nicht komplett ausgeschlossen, diese können an Stellen mit günstigen Temperaturverhältnissen verwendet werden bzw. wo dies aus Designerwägungen heraus angebracht erscheint, z. B. als Sportschaltknauf.

Alternativ können für die EKG-Aufnahme im Fahrzeug Elektroden aus leitfähigem Kunststoff verschiedener Härten (so genannter "Shore"-Faktoren) und Leitfähigkeiten, Textilelektroden (z. B. in Form von in dem Bezugsstoff der Umgebung des Fahrgastraums eingewebten dünnen metallischen Leitungen, also beispielsweise auf den Armlehnen) oder Kapazitivelektroden verwendet werden.

Bei einem weiteren Ausführungsbeispiel der vorliegenden Erfindung kann es sich um ein System zur Erfassung und kontinuierlichen Überwachung ("Monitoring") des EKG eines Kraftfahrzeugfahrers, Zugführers, Piloten usw. handeln. Dieses System kann entweder zur medizinischen Betreuung kardiologischer Patienten, als Fahrerassistenzsystem oder zur Förderung eines gesunden Lebensstils (beispielsweise als sogenanntes "Lifestyle"-Produkt) verwendet werden. Das EKG erlaubt Rückschlüsse auf akute Beeinträchtigungen (z.B. der Fahrleistung) durch Stress, Aggressionen oder das Erkennen einer Gefahrensituation und chronische Krankheitsverläufe, die sich in Rhythmusstörungen oder in einem Herzinfarkt manifestieren. Ein Ausführungsbeispiel der vorliegenden Erfindung kann an die Umgebungsbedingungen in einem Fahrzeug, beispielsweise ein Automobil, angepasst werden. Vor allem entsprechen die Sensoren und Messverfahren empfehlenswerterweise den folgenden Bedingungen: die Beeinträchtigung des Fahrers ist so gering wie möglich gehalten, die Auswahl der Einbauorte für die Sensorik erfolgt im Hinblick auf Qualität der aufzuzeichnenden Messwerte und Fahrerkomfort und bei der Integration von Sensorik sind Sicherheitsaspekte (sicherheitsrelevante Baugruppen, wie z. B. Lenkrad und Rückhaltesystem) im Kfz bzw. Fahrzeug zu berücksichtigen.

Im Gegensatz zu den meisten bekannten Systemen zur Fahrerüberwachung mittels Vitalsensorik (beispielsweise EKG oder Pulsmessung) beachten Ausführungsbeispiele der vorliegenden Erfindung wichtige Aspekte wie beispielsweise, Signalqualität, Abdeckung des Fahrgastraums mit Sensoren, Wahrscheinlichkeit und Häufigkeit der Berührung der Sensoren durch den Fahrer, und Ablenkung des Fahrers durch die Interaktion mit dem Messsystem und eventuell resultierende Beeinträchtigung der Fahrsicherheit.

Während typischerweise die Erfassung des EKGs in einem Kfz bzw. Fahrzeug aufgrund der herausfordernden Randbedingungen bzw. der fehlenden Fahrerakzeptanz, beispielsweise durch Sensoren am Körper, sehr schwer ist, ermöglicht ein Ausführungsbeispiel der vorliegenden Erfindung durch Integration der Sensoren in Bedien- und Ruhelemente eines Kfz und durch die Redundanz der Elektroden eine Erfassung der EKG-Signale mit einer an den jeweiligen Anwendungsfall angepassten Präzision. Die Redundanz der Elektroden, wie sie beispielsweise in den Fig.5-7 gezeigt sind, führt beispielsweise in Kombination mit einer Auswahl und Verarbeitung der Elektrodensignale unter Verwendung der Signalerfassungsvorrichtung gemäß den Fig. 3 und 4 zu besonders guten Ergebnissen, da unter Verwendung der Signalerfassungsvorrichtung die Redundanz der Elektroden zur Verbesserung der Bestimmung des Vitalparameters führt.

Das Anwendungsgebiet der Erfindung liegt im Bereich der präventiven, überwachenden und begleitenden Medizin sowie auf dem Gebiet der Fahrerassistenz und Lifestyle-Applikation (Fitnesscoaching). Ein Vorteil von Ausführungsbeispielen der vorliegenden Erfindung ist, dass die Genauigkeit der Fahrerzustandserkennung durch den Einsatz von innovativen Sensoren, ergonomischer Integration der Sensoren ins Fahrzeuginterieur und vor allem durch eine Erfassung von EKGs mittels redundanten Elektroden mit nachstehender Analyse und Auswahl der besten Signalquelle, wesentlich verbessert wird.

Ein weiterer Vorteil ist, dass ein Ausführungsbeispiel gemäß der vorliegenden Erfindung über wesentlich erweiterte Signalabtastmöglichkeiten und über eine größere Gesamtabtastfläche gegenüber bereits bekannten Sensoranordnung zur Erfassung eines Vitalparameters, verfugt. So werden die Vitalparameter des Kfz-Benutzers mit größerer Zuverlässigkeit als bei anderen bekannten Systemen erfasst, da der Kontakt des Kfz-Benutzers mit den Sensorkomponenten mit größerer Wahrscheinlichkeit geschieht.

Ein weiterer Vorteil ist, dass ein Anbringen der sogenannten Anwendungsteile (Teile, die herkömmlicherweise am Patientenkörper angebracht werden, also beispielsweise Elektroden) am Körper des Fahrers zur Messung der Vitalparameter nicht vorausgesetzt wird.

Weiterhin wird einem Kfz-Benutzer nicht vorgegeben, mit bestimmten Sensorelementen während der Fahrt zwecks Messungen in Berührung zu kommen. Die Messungen erfolgen störungsfrei für den Fahrer.

Weiterhin können die Systemelemente derart in das Interieur und die Bedienelemente des Fahrzeugs integriert werden, dass sie von außen unauffällig bis nicht erkennbar sind bzw. als Designelemente integriert werden.

Obwohl die gezeigten Ausführungsbeispiele einer Signalerfassungsvorrichtung und einer Elektrodenanordnung ausgebildet sind, um ein EKG eines Lebewesens zu erfassen, so ist es auch möglich, dass weitere Ausführungsbeispiele ausgebildet sind um andere Vitalparameter, beispielsweise den Puls eines Lebewesens zu erfassen.

Zusammenfassend lässt sich sagen, dass Ausführungsbeispiele der vorliegenden Erfindung ein Verfahren und eine Vorrichtung, die eine Durchführung von Messungen eines EKG unter Automobilbedingungen ohne Beeinträchtigung eines Kfz-Benutzers mit erhöhter Sensitivität und erhöhter Signalqualität ermöglichen. Dabei werden die Möglichkeiten zur Abtastung des Signals durch mehrere redundante Elektroden im Fahrgastraum wesentlich erweitert.

Das beschriebene Verfahren bzw. die beschriebenen Vorrichtungen sehen die Integration der Vorrichtungen (Signalerfassungsvorrichtung und Elektrodenanordnung) zur Messung des EKG in die Elemente des Innenraums (z. B. Steuerelemente, Bedienelemente, Schaltelemente usw.) vor, mit denen ein Fahrer während der Fahrt in unmittelbaren Kontakt kommt.

Obwohl Ausführungsbeispiele der vorliegenden Erfindung zur Integration in den Innenraum eines Kfz ausgebildet sind. So existieren weiterhin Ausführungsbeispiele, welche ausgebildet sind, um in anderen Fahrzeugen integriert zu werden, welche eine kontinuierliche Überwachung von Vitalparametern des Fahrzeugbenutzers erfordern. Dies können beispielsweise Flugzeuge, Busse usw. sein.

Die erfassten EKGs können optional verrechnet, abgespeichert, analysiert und dem Kfz-Benutzer und/oder dem betreuenden Arzt (im Fall des medizinischen Anwendungsfalls) direkt bzw. das Ergebnis der Auswertung in geeigneter Weise dargestellt werden. Bei Überschreitung der anpassbaren Grenzwerte der Vitalparameter kann eine entsprechende Warnmeldung bzw. Informationsmeldung ausgelöst werden.

Weiterhin ist es möglich, dass für jeden Fahrer ein eigenes Fahrerprofil mit individuellen Schwellwerten definiert wird, um interpersonelle Streuungen zu eliminieren. Anschließend kann anhand des Fahrerprofils und der erfassten Vitalparameter ein individuelles Leistungsprofil (Leistungsreserve) erstellt werden. Mit dem Leistungsprofil kann eine optimale Leistungsschätzung für den Fahrer, abhängig von seinem Gesundheitszustand, durchgeführt werden (beispielsweise eine Schätzung, wie lange der Fahrer noch ohne Gefahr für sich selbst und für andere Verkehrsteilnehmer fahren kann, oder wie lange eine Pause sein sollte).

Die Rolle von Ausführungsbeispielen der vorliegenden Erfindung in einem Kfz bzw. einem anderen Verkehrsmittel liegt in der Unterstützung eines Fahrers bzw. eines Patienten oder in der Warnung des Fahrers bzw. eines Patienten und/oder des betreuenden Arztes, falls der ermittelte Personenzustand eine Gefährdung der Person selbst und anderer Verkehrsteilnehmer darstellt. Mittelfristig kann das System optional weiterentwickelt werden und auch für Diagnosestellungen geeignet sein.

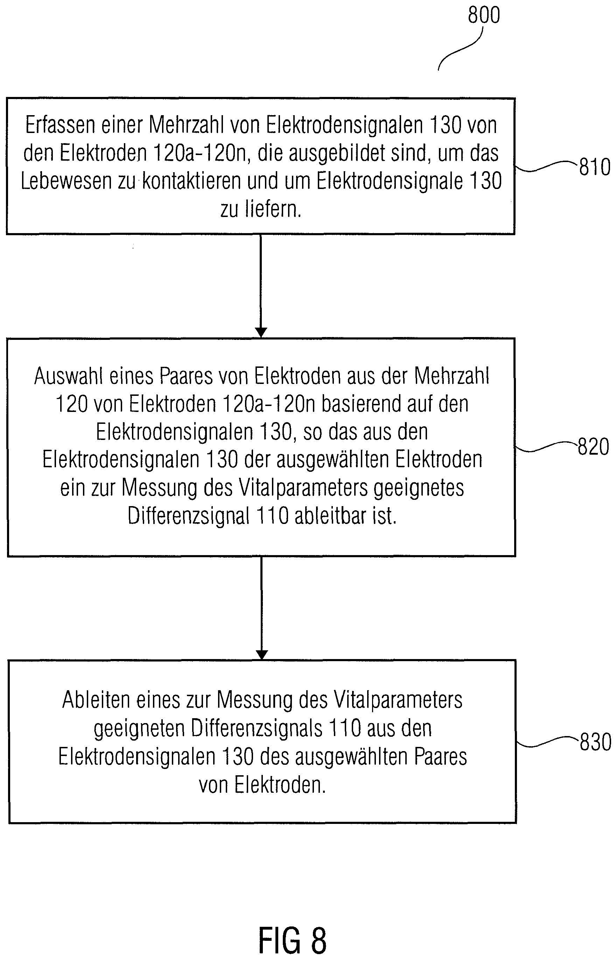

Fig. 8 zeigt ein Flussdiagram eines Verfahrens 800 gemäß einem Ausführungsbeispiel der vorliegenden Erfindung. Das Verfahren 800 zur Erfassung eines Differenzsignals für eine elektrische Messung eines Vitalparameters eines Lebewesens durchgeführt bei einer Elektrodenauswahlvorrichtung 140 unter Verwendung einer Mehrzahl von Elektrodensignalen 130 von einer Mehrzahl 120 von Elektroden 120a-120n umfasst einen ersten Schritt des Anlegens eines sinusförmigen oder modulierten Referenzsignals an eine Referenzelektrode, die in einem Fahrersitz des Kfz integriert ist, und einen zweiten Schritt des Erfassens einer Mehrzahl von Elektrodensignalen 130 aus den Elektroden120a-120n, die ausgebildet sind, um das Lebewesen zu kontaktieren und um Elektrodensignale 130 zu liefern. Weiterhin umfasst das Verfahren 800 einen: dritten Schritt des Auswählens eines Paares von Elektroden aus der Mehrzahl 120 von Elektroden 120a-120n basierend auf den Elektrodensignalen 130, so dass aus den Elektrodensignalen 130 der ausgewählten Elektroden ein zur Messung des Vitalparameters geeignetes Differenzsignal 110 ableitbar ist basierend auf einem Erfassen von, von einzelnen Elektroden aus der Mehrzahl von Elektroden gelieferten Signalen und vergleichen dieser Signale mit dem Referenzsignal, um Elektroden mit bestehendem elektrischen oder kapazitiv gekoppelten Kontakt zu dem Lebewesen zu ermitteln, wobei die Referenzelektrode bei der Auswahl des Paares von Elektroden zur Bestimmung des Differenzsignals unberücksichtigt bleibt. Weiterhin umfasst das Verfahren 800 einen vierten Schritt des Ableitens eines zur Messung des Vitalparameters geeigneten Differenzsignals 110 aus den Elektrodensignalen 130 des ausgewählten Paares von Elektroden.

Das Verfahren 800 kann um alle Merkmalen und Funktionalitäten ergänzt werden, die im Hinblick auf die erfindungsgemäßen Vorrichtungen beschrieben wurden.

## Patentansprüche

1. Signalerfassungsvorrichtung (100; 300; 400) zur Bestimmung eines Differenzsignals (110) für eine elektrische Messung eines Vitalparameters eines Lebewesens, mit folgenden Merkmalen:
einer Mehrzahl (120) von Elektroden (120a-120n), die ausgebildet sind, um das Lebewesen zu kontaktieren und um Elektrodensignale (130) zu liefern; und
einer Elektrodenauswahlvorrichtung (140), die ausgebildet ist, um aus der Mehrzahl (120) von Elektroden (120a-120n) basierend auf den Elektrodensignalen (130) ein Paar von Elektroden zur Bestimmung des Differenzsignals (110) auszuwählen, so dass aus den Elektrodensignalen (130) der ausgewählten Elektroden (120a-120n) ein zur Messung des Vitalparameters geeignetes Differenzsignal (110) ableitbar ist;
wobei die Mehrzahl der Elektroden (120a-120n) in einem Fahrgastraum eines Kraftfahrzeugs (Kfz) integrierbar sind;
wobei die Signalerfassungsvorrichtung ferner eine Referenzelektrode (410) umfasst, die in einem Fahrersitz des Kfz integrierbar ist;
wobei die Elektrodenauswahlvorrichtung (140) ausgebildet ist, um ein sinusförmiges oder moduliertes Referenzsignal (310), das für eine Elektrodendetektion verwendet wird, an die Referenzelektrode (410) anzulegen, und um die Referenzelektrode (410) bei der Auswahl des Paares von Elektroden zur Bestimmung des Differenzsignals (110) unberücksichtigt zu lassen; und
wobei die Elektrodenauswahlvorrichtung (140) ausgebildet ist, um von einzelnen Elektroden (120a-120n) aus der Mehrzahl (120) von Elektroden (120a-120n) gelieferte Signale (130a-130n) zu erfassen und diese Signale (130a-130n) mit dem Referenzsignal (310) zu vergleichen, um Elektroden (120a-120n) mit bestehendem elektrischen oder kapazitiv gekoppelten Kontakt zu dem Lebewesen zu ermitteln und für eine Messung des Differenzsignals (110) zu aktivieren.

2. Signalerfassungsvorrichtung (100; 300; 400) gemäß Anspruch 1,
bei der die Elektrodenauswahlvorrichtung (140) ausgebildet ist, um im Rahmen einer Auswahl eines Paares von Elektroden für die Messung des Vitalparameters ein erstes Differenzsignal (110) eines ersten Paares von Elektroden aus der Mehrzahl (120) von Elektroden (120a-120n) mit einem zweiten Differenzsignal (110) eines zweiten Paares von Elektroden aus der Mehrzahl (120) von Elektroden (120a-120n) zu vergleichen, und
um das Paar von Elektroden zur Bestimmung des Differenzsignals (110) für die elektrische Messung des Vitalparameters auszuwählen, das im Rahmen der Auswahl das größte Differenzsignal (110) aufweist.

3. Signalerfassungsvorrichtung (100; 300; 400) gemäß einem der Ansprüche 1 bis 2,
die ferner eine Speichervorrichtung umfasst, die ausgebildet ist, um eine Dauer der Kontaktierung der Elektroden (120a-120n) aus der Mehrzahl (120) der Elektroden (120a-120n) mit dem Lebewesen zu erfassen, und
um eine Kontaktierungswahrscheinlichkeit der Elektroden (120a-120n), basierend auf der Dauer der Kontaktierung der Elektroden (120a-120n) mit dem Lebewesen abzuleiten.

4. Signalerfassungsvorrichtung (100; 300; 400) gemäß Anspruch 3, bei der die Mehrzahl (120) der Elektroden eine erste Gruppe von Elektroden (610) und eine zweite Gruppe von Elektroden (620) umfasst;
wobei die erste Gruppe von Elektroden (610) einer ersten Körperhälfte des Kfz-Benutzers zugeordnet ist;
wobei die zweite Gruppe von Elektroden (620) einer zweiten Körperhälfte des Kfz-Benutzers zugeordnet ist;
wobei die erste Gruppe von Elektroden (620) angeordnet ist, um die erste Körperhälfte des Kfz-Benutzers zu kontaktieren; und
wobei die zweite Gruppe von Elektroden (620) angeordnet ist, um die zweite Körperhälfte des Kfz-Benutzers zu kontaktieren; und
bei der die Elektrodenauswahlvorrichtung (130) ausgebildet ist, um das Paar von Elektroden (120a-120n) zur Bestimmung des Differenzsignals (110) für die elektrische Messung eines Vitalparameters so auszuwählen, dass eine erste Elektrode (120a; 210) des Paares aus der ersten Gruppe von Elektroden (610) stammt und eine zweite Elektrode (120b; 220) des Paares aus der zweiten Gruppe von Elektroden (620) stammt.

5. Signalerfassungsvorrichtung (400) gemäß einem der vorhergehenden Ansprüche,
wobei mindestens eine erste Elektrode (210) aus der Mehrzahl der Elektroden an oder in einem Bedienelement (230) des Kfz anordenbar ist; und
wobei mindestens eine zweite Elektrode (220) aus der Mehrzahl der Elektroden an oder in einem Ruheelement (240) des Kfz anordenbar ist.

6. Signalerfassungsvorrichtung (400) gemäß Anspruch 5,
bei der die erste Elektrode (210) aus der Mehrzahl der Elektroden so angeordnet ist, um eine erste Hand oder einen ersten Arm des Kfz-Benutzers zu kontaktieren; und
die zweite Elektrode (220) aus der Mehrzahl der Elektroden so angeordnet ist, um eine zweite Hand oder einen zweiten Arm des Kfz-Benutzers zu kontaktieren.

7. Signalerfassungsvorrichtung (400) gemäß einem der Ansprüche 1 bis 6,
wobei die Referenzelektrode in dem Fahrersitz des Kfz angeordnet ist, um den Kfz-Benutzer kapazitiv zu koppeln.

8. Signalerfassungsvorrichtung (400) gemäß einem der Ansprüche 1 bis 7,
bei der mindestens eine dritte Elektrode aus der Mehrzahl der Elektroden an oder in einem Sicherheitselement des Kfz anordenbar ist.

9. Signalerfassungsvorrichtung (400) gemäß einem der Ansprüche 1 bis 8,
bei der mindestens eine Elektrode aus der Mehrzahl der Elektroden eine metallisierte Kunststoffschicht umfasst.

10. Signalerfassungsvorrichtung (400) gemäß einem der Ansprüche 1 bis 9,
bei der mindestens eine Elektrode aus der Mehrzahl der Elektroden eine Schicht aus leitfähigem Kunststoff umfasst.

11. Signalerfassungsvorrichtung (400) gemäß einem der Ansprüche 1 bis 10,
bei der mindestens eine Elektrode aus der Mehrzahl der Elektroden als eine Textilelektrode ausgebildet ist.

12. Signalerfassungsvorrichtung (400) gemäß einem der Ansprüche 1 bis 11,
bei der mindestens eine der Elektroden aus der Mehrzahl der Elektroden als eine Kapazitivelektrode ausgebildet ist, um den Kfz-Benutzer kapazitiv zu koppeln.

13. Verfahren (800) zur Bestimmung eines Differenzsignals (110) für eine elektrische Messung eines Vitalparameters eines Lebewesens unter Verwendung einer Mehrzahl von Elektrodensignalen (130) von einer Mehrzahl (120) von Elektroden (120a-120n), wobei die Mehrzahl der Elektroden (120a-120n) in einem Fahrgastraum eines Kraftfahrzeugs (Kfz) integriert sind, mit folgenden Schritten:
a) Anlegen, mittels einer Elektrodenauswahlvorrichtung (140), eines sinusförmigen oder modulierten Referenzsignals, das für eine Elektrodendetektion verwendet wird, (310) an eine Referenzelektrode (410), die in einem Fahrersitz des Kfz integriert ist;
b) Erfassen, mittels der Elektrodenauswahlvorrichtung (140), einer Mehrzahl von Elektrodensignalen (130) von den Elektroden (120a-120n), die ausgebildet sind, um das Lebewesen zu kontaktieren und um Elektrodensignale (130) zu liefern;
c) Auswahl, mittels der Elektrodenauswahlvorrichtung (140), eines Paares von Elektroden aus der Mehrzahl (120) von Elektroden (120a-120n) basierend auf den Elektrodensignalen (130), so dass aus den Elektrodensignalen (130) der ausgewählten Elektroden ein zur Messung des Vitalparameters geeignetes Differenzsignal ableitbar ist, basierend auf einem Erfassen von, von einzelnen Elektroden (120a-120n) aus der Mehrzahl (120) von Elektroden (120a-120n) gelieferten Signalen (130a-130n) und vergleichen dieser Signale (130a-130n) mit dem Referenzsignal (310), um Elektroden (120a-120n) mit bestehendem elektrischen oder kapazitiv gekoppelten Kontakt zu dem Lebewesen zu ermitteln, wobei die Referenzelektrode (410) bei der Auswahl des Paares von Elektroden zur Bestimmung des Differenzsignals (110) unberücksichtigt bleibt; und
d) Ableiten, mittels der Elektrodenauswahlvorrichtung (140), eines zur Messung des Vitalparameters geeigneten Differenzsignals (110) aus den Elektrodensignalen (130) des ausgewählten Paares von Elektroden (120a-120n).

## Claims

1. A signal detecting device (100; 300; 400) for determining a difference signal (110) for an electrical measurement of a vital parameter of a living being, comprising:
a plurality (120) of electrodes (120a-120n) configured to contact the living being and provide electrode signals (130); and
an electrode selecting device (140) configured to select a pair of electrodes for determining the difference signal (110) from the plurality (120) of electrodes (120a-120n) based on the electrode signals (130) so that a difference signal (110) suitable for measuring the vital parameter is derivable from the electrode signals (130) of the selected electrodes (120a-120n);
wherein the plurality of electrodes (120a-120n) are integrable in a passenger area of a motorcar (MC);
wherein the signal detecting device further includes a reference electrode (410) being integrable in a driver's seat of the MC;
wherein the electrode selecting device (140) is configured to apply a sinusoidal or modulated reference signal (310) used for electrode detection to the reference electrode (410) and to disregard the reference electrode (410) when selecting the pair of electrodes for determining the difference signal (110); and
wherein the electrode selecting device (140) is configured to detect signals (130a-130n) supplied by individual electrodes (120a-120n) of the plurality (120) of electrodes (120a-120n) and to compare these signals (130a-130n) to the reference signal (310) to determine electrodes (120a-120n) having existing electrical or capacitively coupled contact with the living being and to activate them for measurement of the difference signal (110).

2. The signal detecting device (100; 300; 400) in accordance with claim 1,
wherein the electrode selecting device (140) is configured to compare, when selecting a pair of electrodes for measuring the vital parameter, a first difference signal (110) of a first pair of electrodes from the plurality (120) of electrodes (120a-120n) to a second difference signal (110) of a second pair of electrodes from the plurality (120) of electrodes (120a-120n), and
to select that pair of electrodes for determining the difference signal (110) for the electrical measurement of the vital parameter, comprising, when selecting, the greatest difference signal (110).

3. The signal detecting device (100; 300; 400) in accordance with one of claims 1 to 2,
further including a storage device configured to detect a duration of contacting of the electrodes (120a-120n) from the plurality (120) of electrodes (120a-120n) to the living being, and
to derive a contacting probability of the electrodes (120a-120n) based on the duration of the contacting of the electrodes (120a-120n) to the living being.

4. The signal detecting device (100; 300; 400) in accordance with claim 3, wherein the plurality (120) of electrodes include a first group of electrodes (610) and a second group of electrodes (620);
wherein the first group of electrodes (610) is associated to a first body half of the MC user;
wherein the second group of electrodes (620) is associated to a second body half of the MC user;
wherein the first group of electrodes (620) is arranged to contact the first body half of the MC user; and
wherein the second group of electrodes (620) is arranged to contact the second body half of the MC user; and
wherein the electrode selecting device (130) is configured to select the pair of electrodes (120a-120n) for determining the difference signal (110) for the electrical measurement of a vital parameter such that a first electrode (120a; 210) of the pair is from the first group of electrodes (610) and a second electrode (120b; 220) of the pair is from the second group of electrodes (620).

5. The signal detecting device (400) in accordance with one of the preceding claims:
wherein at least a first electrode (210) from the plurality of electrodes can be arranged at or in an operating element (230) of the MC; and
wherein at least a second electrode (220) from the plurality of electrodes can be arranged at or in a rest element (240) of the MC.

6. The signal detecting device (400) in accordance with claim 5,
wherein the first electrode (210) from the plurality of electrodes is arranged to contact a first hand or a first arm of the MC user; and
the second electrode (220) from the plurality of electrodes is arranged to contact a second hand or a second arm of the MC user.

7. The signal detecting device (400) in accordance with one of claims 1 to 6,
wherein the reference electrode is arranged in the driver's seat of the MC so as to couple the MC user capacitively.

8. The signal detecting device (400) in accordance with one of claims 1 to 7,
wherein at least a third electrode from the plurality of electrodes can be arranged at or in a security element of the MC.

9. The signal detecting device (400) in accordance with one of claims 1 to 8,
wherein at least one electrode from the plurality of electrodes includes a metallized plastic layer.

10. The signal detecting device (400) in accordance with one of claims 1 to 9,
wherein at least one electrode from the plurality of electrodes includes a layer made of conductive plastic.

11. The signal detecting device (400) in accordance with one of claims 1 to 10,
wherein at least one electrode from the plurality of electrodes is implemented to be a textile electrode.

12. The signal detecting device (400) in accordance with one of claims 1 to 11,
wherein at least one of the electrodes from the plurality of electrodes is implemented to be a capacitive electrode so as to couple the MC user capacitively.

13. A method (800) for determining a difference signal (110) for an electrical measurement of a vital parameter of a living being using a plurality of electrode signals (130) from a plurality (120) of electrodes (120a-120n), wherein the plurality of electrodes (120a-120n) are integrated in a passenger area of a motorcar (MC), comprising:
a) applying, by means of an electrode selecting device (140), a sinusoidal or modulated reference signal used for electrode detection (310) to a reference electrode (410) integrated in a driver's seat of the MC;
b) detecting, by means of the electrode selecting device (140), a plurality of electrode signals (130) from the electrodes (120a-120n) configured to contact the living being and provide electrode signals (130);
c) selecting, by means of an electrode selecting device (140), a pair of electrodes from the plurality (120) of electrodes (120a-120n) based on the electrode signals (130) such that a difference signal suitable for measuring the vital parameter is derivable from the electrode signals (130) of the selected electrodes, based on detecting signals (130a-130n) supplied by individual electrodes (120a-120n) from the plurality (120) of electrodes (120a-120n) and comparing these signals (130a-130n) to the reference signal (310) to determine electrodes (120a-120n) having existing electrical or capacitively coupled contact with the living being, the reference electrode (410) being disregarded when selecting the pair of electrodes for determining the difference signal (110); and
d) deriving, by means of the electrode selecting device (140), a difference signal (110) suitable for measuring the vital parameter from the electrode signals (130) of the selected pair of electrodes (120a-120n).

## Revendications

1. Dispositif de détection de signal (100; 300; 400) pour déterminer un signal différentiel (110) pour une mesure électrique d'un paramètre vital d'un être vivant, aux caractéristiques suivantes:
une pluralité (120) d'électrodes (120a à 120n) qui sont conçues pour entrer en contact avec l'être vivant et pour fournir des signaux d'électrode (130); et
un dispositif de sélection d'électrodes (140) qui est conçu pour sélectionner parmi la pluralité (120) d'électrodes (120a à 120n), sur base des signaux d'électrode (130), une paire d'électrodes pour déterminer le signal différentiel (110) de sorte qu'à partir des signaux d'électrode (130) des électrodes sélectionnées (120a à 120n) puisse être dérivé un signal différentiel (110) convenant pour la mesure du paramètre vital;
dans lequel la pluralité d'électrodes (120a à 120n) peuvent être intégrées dans un habitacle d'un véhicule automobile;
dans lequel le dispositif de détection de signal comporte par ailleurs une électrode de référence (410) qui peut être intégrée dans le siège de conducteur du véhicule;
dans lequel le dispositif de sélection d'électrodes (140) est conçu pour appliquer à l'électrode de référence (410) un signal de référence sinusoïdal ou modulé (310) qui est utilisé pour une détection d'électrodes et pour ne pas prendre en compte l'électrode de référence (410) lors de la sélection de la paire d'électrodes pour la détermination du signal différentiel (110); et
dans lequel le dispositif de sélection d'électrodes (140) est conçu pour détecter des signaux (130a à 130n) fournis par des électrodes individuelles (120a à 120n) de la pluralité (120) d'électrodes (120a à 120n) et pour comparer ces signaux (130a à 130n) avec le signal de référence (310) pour déterminer les électrodes (120a à 120n) existantes à contact électrique avec ou couplé de manière capacitive à l'être vivant et pour les activer pour une mesure du signal différentiel (110).

2. Dispositif de détection de signal (100; 300; 400) selon la revendication 1,
dans lequel le dispositif de sélection d'électrodes (140) est conçu pour comparer, dans le cadre d'une sélection d'une paire d'électrodes pour la mesure du paramètre vital, un premier signal différentiel (110) d'une première paire d'électrodes de la pluralité (120) d'électrodes (120a à 120n) avec un deuxième signal différentiel (110) d'une deuxième paire d'électrodes de la pluralité (120) d'électrodes (120a à 120n), et
pour sélectionner, pour déterminer le signal différentiel (110) pour la mesure électrique du paramètre vital, la paire d'électrodes qui, dans le cadre de la sélection, présente le plus grand signal différentiel (110).

3. Dispositif de détection de signal (100; 300; 400) selon l'une des revendications 1 à 2,
qui comporte par ailleurs un dispositif de mémoire qui est conçu pour détecter une durée d'entrée en contact des électrodes (120a à 120n) de la pluralité (120) des électrodes (120a à 120n) avec l'être vivant, et
pour dériver une probabilité d'entrée en contact des électrodes (120a à 120n) sur base de la durée d'entrée en contact des électrodes (120a à 120n) avec l'être vivant.

4. Dispositif de détection de signal (100; 300; 400) selon la revendication 3, dans lequel la pluralité (120) des électrodes comporte un premier groupe d'électrodes (610) et un deuxième groupe d'électrodes (620);
dans lequel le premier groupe d'électrodes (610) est associé à une première moitié du corps de l'utilisateur du véhicule automobile;
dans lequel le deuxième groupe d'électrodes (620) est associé à une deuxième moitié du corps de l'utilisateur du véhicule automobile;
dans lequel le premier groupe d'électrodes (620) est disposé de manière à entrer en contact avec la première moitié du corps de l'utilisateur du véhicule automobile; et
dans lequel le deuxième groupe d'électrodes (620) est disposé de manière à entrer en contact avec la deuxième moitié du corps de l'utilisateur du véhicule automobile; et
dans lequel le dispositif de sélection d'électrodes (130) est conçu pour sélectionner la paire d'électrodes (120a à 120n) pour la détermination du signal différentiel (110) pour la mesure électrique d'un paramètre vital de sorte qu'une première électrode (120a; 210) soit de la paire du premier groupe d'électrodes (610) et qu'une deuxième électrode (120b, 220) soit de la paire du deuxième groupe d'électrodes (620).

5. Dispositif de détection de signal (400) selon l'une des revendications précédentes,
dans lequel au moins une première électrode (210) de la pluralité des électrodes peut être disposée sur ou dans un élément de commande (230) du véhicule automobile; et
dans lequel au moins une deuxième électrode (220) de la pluralité des électrodes peut être disposée sur ou dans un élément de repos (240) du véhicule automobile.

6. Dispositif de détection de signal (400) selon la revendication 5,
dans lequel la première électrode (210) de la pluralité des électrodes est disposée de manière à entrer en contact avec une première main ou un premier bras de l'utilisateur du véhicule automobile; et
la deuxième électrode (220) de la pluralité des électrodes est disposée de manière à entrer en contact avec une deuxième main ou un deuxième bras de l'utilisateur du véhicule automobile.

7. Dispositif de détection de signal (400) selon l'une des revendications 1 à 6,
dans lequel l'électrode de référence est disposée dans le siège du conducteur du véhicule automobile pour coupler de manière capacitive l'utilisateur du véhicule automobile.

8. Dispositif de détection de signal (400) selon l'une des revendications 1 à 7,
dans lequel au moins une troisième électrode de la pluralité d'électrodes peut être disposée sur ou dans un élément de sécurité du véhicule automobile.

9. Dispositif de détection de signal (400) selon l'une des revendications 1 à 8,
dans lequel l'au moins une électrode de la pluralité d'électrodes comporte une couche de matière plastique métallisée.

10. Dispositif de détection de signal (400) selon l'une des revendications 1 à 9,
dans lequel l'au moins une électrode de la pluralité d'électrodes comporte une couche de matière plastique conductrice.

11. Dispositif de détection de signal (400) selon l'une des revendications 1 à 10,
dans lequel l'au moins une électrode de la pluralité d'électrodes est réalisée sous forme d'électrode textile.

12. Dispositif de détection de signal (400) selon l'une des revendications 1 à 11,
dans lequel au moins une des électrodes de la pluralité d'électrodes est formée sous forme d'électrode capacitive pour coupler de manière capacitive l'utilisateur du véhicule automobile.

13. Procédé (800) pour déterminer un signal différentiel (110) pour une mesure électrique d'un paramètre vital d'un être vivant à l'aide d'une pluralité de signaux d'électrodes (130) d'une pluralité (120) d'électrodes (120a à 120n), où la pluralité d'électrodes (120a à 120n) 120n) sont intégrées dans un habitacle d'un véhicule automobile, aux étapes suivantes consistant à:
a) appliquer, au moyen d'un dispositif de sélection d'électrodes (140), un signal de référence sinusoïdal ou modulé qui est utilisé pour une détection d'électrodes (310), à une électrode de référence (410) qui est intégrée dans un siège de conducteur du véhicule automobile;
b) détecter, au moyen du dispositif de sélection d'électrodes (140), une pluralité de signaux d'électrode (130) des électrodes (120a à 120n) qui sont conçues pour entrer en contact avec l'être vivant et pour fournir des signaux d'électrode (130);
c) sélectionner, au moyen du dispositif de sélection d'électrodes (140), une paire d'électrodes parmi la pluralité (120) d'électrodes (120a à 120n), sur base des signaux d'électrode (130), de sorte qu'à partir des signaux d'électrode (130) des électrodes sélectionnées puisse être dérivé un signal différentiel convenant pour la mesure du paramètre vital, sur base d'une détection de signaux (130a à 130n) fournis par des électrodes individuelles (120a à 120n) de la pluralité (120) d'électrodes (120a à 120n) et comparer ces signaux (130a à 130n) avec le signal de référence (310) pour déterminer des électrodes (120a à 120n) existantes à contact électrique avec ou couplé de manière capacitive à l'être vivant, l'électrode de référence (410) n'étant pas pris en compte lors de la sélection de la paire d'électrodes pour la détermination du signal différentiel (110); et
d) dériver, au moyen du dispositif de sélection d'électrodes (140), un signal différentiel (110) convenant pour mesurer le paramètre vital à partir des signaux d'électrodes (130) de la paire d'électrodes sélectionnée (120a à 120n).
